# EUROPEAN PATENT APPLICATION

(11) **EP 4 368 095 A1**
(43) Date of publication of application: **15.05.2024**
(21) Application number: 22383086.0
(22) Date of filing: 11.11.2022
(51) Int. Cl.: A61B 1/00

(54) **A DEVICE FOR ASSISTING TRANSLATIONAL MOVEMENT OF AN ENDOSCOPE THROUGH THE DIGESTIVE TRACT**

(71) Applicant: Universitat Politècnica de València, 46022 Valencia (ES); Fundación Para la Investigación del Hospital Universitario y Politécnico La Fe de la Comunidad Valenciana, 46026 Valencia (ES)
(72) Inventor: PONS BELTRÁN, Vicente, 46026 Valencia (ES); SÁNCHEZ DÍAZ, Carlos, 46022 Valencia (ES); SANTONJA GIMENO, Alberto Vicente, 46022 Valencia (ES); VIDAURRE GARAYO, Ana, 46022 Valencia (ES); ZAZO MANZANEQUE, Roberto, 46022 Valencia (ES); LORENZO-ZÚÑIGA GARCÍA, Vicente María, 46026 Valencia (ES)
(74) Representative: ZBM Patents - Zea, Barlocci & Markvardsen

(57) **Abstract**

Disclosed herein is a device (1,1') for assisting translational movement of an endoscope (2) through the digestive tract (T), comprising:
- a fixed radial expansion balloon (3) mounted on a fixed support bushing (4) to be immobilized on the endoscope (2);
- a movable radial expansion balloon (5) mounted on a movable support bushing (6) to be mounted floating on the endoscope (2); and
- an axial actuator (7, 7') attached between the movable radial expansion balloon (5) and a distal end (2a) of the endoscope (2), the axial actuator comprising:
- a first unit of axial expansion chambers (8) attached to the movable support bushing (6);
- a second unit of axial expansion chambers (10) attached to a movable distal bushing (11); and
- a wire (12) attached between the movable support bushing (6) and the movable distal bushing (11).

## Description

The present disclosure relates to a device and a system for assisting translational movement of an endoscope through the digestive tract.

### BACKGROUND

In the field of clinical gastroenterology, digestive tract pathologies occupy the most important volume. From the oesophagus to the rectum there are more than ten metres of digestive tract where a multitude of pathologies can occur. Access to the digestive tract in a minimally invasive manner using the natural orifices, mouth and anus, has been vital for the diagnosis and handling of this large group of diseases.

The development of digestive endoscopy has facilitated such access and enabled the development of therapeutic actions otherwise unimaginable. Many advances have been made in the field of digestive endoscopy, which has made it possible to effectively act in the most proximal and distal portions of the digestive tract.

An endoscopy is a technique for visual exploration on a body cavity, e.g. the small intestine, performed using an endoscope. A conventional endoscope apparatus comprises an endoscope, that is a long, thin insertion tube to be inserted into a body cavity, and connected to a control handle which remains outside the patient's body, to be manipulated by a user, e.g. a doctor. The endoscope includes internal canals for the passage of air, water, surgical instruments, electrical cables, optical fiber, a light, a video camara, and the like, wherein the canals extend from a proximal end of the endoscope (closest to the control handle) up to a distal end thereof.

Upper endoscopy or gastroscopy makes it possible to reach without difficulty and with good acceptance by the patient the proximal structures of the digestive tract: oesophagus, stomach and first and second duodenal portions. This has led to the development of real minimally invasive endoscopic surgery.

Something similar occurs with the colon, the sigmoid colon and the rectum, which are the most distal portions of the digestive tract using colonoscopy. Even with this same technique it is possible to skillfully reach the most distal portion of the small intestine, the terminal ileum.

There has always been an area of the digestive tract which, on the other hand is the longest (about seven metres) that is difficult to access both anterograde (mouth) and retrograde (anal). In recent years, the access to this portion of the digestive tract has been facilitated with the use of well-known techniques, such as called "single-balloon enteroscopy", "double-balloon enteroscopy" and "spiral enteroscopy", among others.

For example, EP1726250 relates to a double-balloon endoscope system, formed by two concentric tubes, at the end of which there are respective inflatable balloons. By inflating one or another of the balloons, the digestive tract is widened, and in this moment the other tube, via the end with the balloon not inflated at that time, move forward. With this movement between both concentric tubes, rhythmic with the inflation or deflation of the balloons, the endoscope is made to advance through the intestinal tract. This simple movement a priori involves certain risks due to uncontrolled advance within the digestive tract and the lack of knowledge of how the intestine walls are constituted, for which reason said system and method involve numerous difficulties and drawbacks which make its execution problematic.

EP2243417 of the same applicants as the present disclosure, discloses a device for translational movement of an endoscope through the digestive tract, e.g. the small intestine, comprising pneumatic translational movement means and electronic control means that enable the automated and coordinated method of the translational movement means. The translational movement means comprises a fixed radial expansion balloon immobilized on the endoscope; a movable radial expansion balloon mounted floating on the endoscope, wherein the two radial expansion balloons can be radially expanded for its fixing to the digestive tract; and at least one bellows-shaped axial expansion chamber connected by a first end to the proximities of the distal end of advance of the body of the endoscope, and which is secured by a second end to the movable radial expansion balloon.

With the movable radial expansion balloon inflated and thus fixed to the digestive tract, the bellows-shaped axial expansion chamber is inflated in order to displace the movable radial expansion balloon backwards (i.e., towards the fixed radial expansion balloon and in the opposite direction of the advance of the endoscope) to cause the retraction of a portion of the digestive tract and, therefore, the relative advance of the endoscope with respect to the retracted digestive tract.

However, as *in vitro* test and *in vivo* test have showed, this kind of device using such a bellows-shaped axial expansion chamber, may not always be efficient to retract the digestive tract, because in some cases the exerted force is not sufficient to overcome the force exerted by the mesentery.

Furthermore, when the bellows-shaped axial expansion chamber is inflated, in practice, it expands not only in the axial direction, but also in the radial direction. As a result, not all the exerted force is applied to move the movable radial expansion balloon backward, and thus there is a risk that only a short portion of the digestive tract is retracted on the endoscope. Hence, in some cases a very small advance distance, e.g. between 5 and 6 mm, is achieved to displace the endoscope through the digestive tract in each cycle.

In addition, when the bellows-shaped axial expansion chamber is deflated, the exerted force may not be enough to return the movable radial expansion balloon forwards (i.e., towards the distal part of the endoscope).

Consequently, there is a need to provide an improved device for translational movement of an endoscope though the digestive tract, e.g. the small intestine, which is capable of providing a greater backward force for retracting a larger portion of the digestive tract on the endoscope, thus indirectly providing a greater advance distance of the endoscope, in each cycle; and also providing an easier and more effective access of the endoscope through the digestive tract, thus reducing the patient discomfort.

It is also desirable to provide a device for automated translational movement of an endoscope designed with a simple structural configuration capable to be adapted to a conventional endoscope.

### SUMMARY

According to one aspect of the present disclosure, a device for assisting translational movement of an endoscope through the digestive tract is provided. The device comprises:
- a fixed radial expansion balloon mounted on a fixed support bushing to be immobilized on the endoscope;
- a movable radial expansion balloon mounted on a movable support bushing to be mounted floating on the endoscope;
   wherein the fixed radial expansion balloon and the movable radial expansion balloon are configured to contact an inner wall of the digestive tract in an expanded position; and
- an axial actuator to be attached between the movable radial expansion balloon and a distal end of the endoscope, wherein the axial actuator comprises:
   - a first unit of axial expansion chambers attached at one end to the movable support bushing of the movable radial expansion balloon;
   - a second unit of axial expansion chambers attached at one end to a movable distal bushing, the movable distal bushing to be mounted floating on the endoscope in a position distal with respect to the first and second units of axial expansion chambers; and
   - a wire attached by respective opposite ends between the movable support bushing of the movable radial expansion balloon and the movable distal bushing;
   and wherein the first unit of axial expansion chambers and the second unit of axial expansion chambers are configured to be synchronously extended and contracted to move axially the movable support bushing of the movable radial expansion balloon with alternate forward and backward movements, along a working distance between
   - a distal position, wherein the first unit of axial expansion chambers is contracted and the second unit of axial expansion chambers is extended, and
   - a proximal position, wherein the first unit of axial expansion chambers is extended and the second unit of axial expansion chambers is contracted.

The first unit and the second unit of axial expansion chambers are configured to actuate in an alternate manner, that is, when the first unit is extended (inflated), the second unit is contracted (deflated) and vice versa.

In the present disclosure by "endoscope" it is understood the insertion tube of a conventional endoscope apparatus.

In the present disclosure, the term "proximal" refers to the portion of the endoscope closest to the handle control of a conventional endoscope apparatus, and the term "distal" refers to the end portion inserted into the patient's body (i.e., toward and away from the user, e.g. a doctor, respectively).

In the present disclosure, by "forwards" it is meant a movement in distal direction (i.e., in the advance direction of the endoscope); and by "backwards" it is meant a movement in proximal direction (i.e., opposite to the advance direction of the endoscope).

In the present disclosure, the digestive tract refers to the oesophagus, stomach and the whole small intestine, as well as the rectum and large intestine until covering all the small intestine. The device of the present disclosure will be very advisable, for example, in the advance of the endoscope through the small intestine due to the fact that said tract is of greater length and more tortuous path, which may further hinder the passage of the endoscope therethrough. For this reason, throughout the present disclosure, there is talk of insertion of the endoscope through the small intestine, but this does not limit its use to any part of the digestive tract.

In the present disclosure, by "radial expansion balloon" it is meant that said balloon, when expanded (e.g. by pneumatic actuation through air or gas or by hydraulic actuation via any liquid fluid), is configured to achieve its enlargement or expansion in radial direction with respect to the endoscope body, i.e., in the direction of radius of said endoscope. On the other hand, by "axial expansion chamber" it is meant that said chamber, when extended, is configured to achieve its enlargement or extension in axial direction with respect to the endoscope body, i.e., in the direction of the axis of the endoscope.

In the present disclosure, a "bushing" is understood as a type of cylindrical or annular support configured to be mounted on or around a shaft, in this case attached to the tubular body of a conventional endoscope. There are two types of bushings described here depending on the function thereof. One type is a fixed bushing configured to be immobilized in a predetermined position on the endoscope body; and another type is a movable bushing configured to be mounted floating on the endoscope body and capable to slide in the axial direction.

Examples of the device disclosed herein may be operated such that:
- First, when the movable radial expansion balloon is placed in the distal position, the movable radial expansion balloon is inflated to contact an inner wall of the digestive tract, and the fixed radial expansion balloon is deflated, and then the first unit of axial expansion chambers is inflated and the second unit of axial expansion chambers is deflated synchronously. Consequently, the first unit of axial expansion chambers propels the movable radial expansion balloon backwards (i.e., in proximal direction) along the working distance to reach the proximal position, and in turn the wire pulls the movable distal bushing backwards. During the backward displacement of the movable radial expansion balloon, a portion of the digestive tract is retracted on the endoscope, thus enabling the indirect advance of the endoscope though the digestive tract.
- After that, the fixed radial expansion balloon is inflated for retaining the portion of digestive tract previously retracted, and the movable radial expansion balloon is deflated, and then the second unit of axial expansion chambers is inflated and the first unit of axial expansion chambers is deflated synchronously. Consequently, the second unit of axial expansion chambers propels the movable distal bushing forwards (i.e., in distal direction), and in turn the wire pulls the movable radial expansion balloon forwards to reach the distal position again. Then, the movable expansion balloon is inflated and the fixed expansion balloon is deflated again; and this cycle is repeated successively for retracting a next portion of digestive tract.

Thanks to the alternate and coordinate movement of the first unit of axial expansion chambers and the second unit of axial expansion chambers, a greater backward force for retracting a larger portion of the digestive tract on the endoscope is achieved with respect to the prior art, thus providing a greater advance distance of the endoscope through the digestive tract, e.g. the small intestine, in each cycle, and especially to overcome the force exerted by the mesentery.

Furthermore, a greater forward force to return the movable radial expansion balloon to the distal position is achieved, in each cycle.

Thus, the device of the present disclosure provides an easy and reliable use thereof for implementing enteroscopies, that is complete explorations of the digestive tract, in particular of the small intestine, and reducing the patient discomfort. Therefore, scanning times are reduced, the number of complete enteroscopies is increased, and the learning curve for new professionals is reduced due to the high degree of automation.

Moreover, the device of the present disclosure may be mounted on a conventional endoscope, thus reducing the manufacturing and maintenance costs.

Furthermore, the device of the present disclosure may be adapted to endoscopes of any diameter.

One function of the wire is to link the second unit of axial expansion chambers to the movable support bushing, such that it exerts a force on this bushing when it is inflated, that is, a force caused by a positive fluid pressure. The distal movement of the movable expansion balloon is therefore also caused by exerting a positive fluid pressure, like its proximal movement, and it does not rely only on the negative fluid pressure associated with the deflation of the first unit of axial expansion chambers.

Furthermore, the wire also contributes in deflating the chambers of one unit while the other unit is inflated, such as to better synchronize the operation of the two units, wherein the first and second units complement each other, thus obtaining an optimal alternate forward and backward movements of the movable radial expansion balloon along the working distance.

In some embodiments, the device may further comprise a fixed intermediate bushing arranged between the first and second units of axial expansion chambers, and to be immobilized on the endoscope: the first unit of axial expansion chambers may thus be attached between the movable support bushing and the fixed intermediate bushing, and the second unit of axial expansion chambers may be attached between the fixed intermediate bushing and the movable distal bushing.

In other embodiments of the device, the fixed intermediate bushing may be omitted, and the first unit of axial expansion chambers may then be attached between the movable support bushing and the fixed support bushing, while the second unit of axial expansion chambers may be attached between the fixed support bushing and the movable distal bushing.

In examples of the present disclosure, the working distance of the movable radial expansion balloon may be comprised between 3 and 15 cm, for example between 5 and 10 cm, and preferably may be approximately 6 cm. Therefore, a larger portion of the digestive tract may be retracted on the endoscope in each cycle, in comparison with known devices using a bellows-shaped axial expansion chamber of the prior art, wherein only a considerably small retraction distance of 5 to 6 mm was achieved, as explained above.

According to one example, each axial expansion chamber of the first unit and the second unit may comprise a tubular shaped body.

The tubular shape allows each of the axial expansion chambers to actuate as a piston, thus improving the propulsion effect for pushing and pulling the movable radial expansion balloon, backwards and forwards, according to the coordinated extension and contraction movements of the respective first unit and second unit of axial expansion chambers, as explained above.

In one preferred example, each of the first unit of axial expansion chambers and the second unit of axial expansion chambers comprise three axial expansion chambers arranged parallel to each other and around the endoscope.

It is noted that during the advance of the endoscope, for example through curved sections of the small intestine, uncontrolled rotation of the movable support bushing supporting the movable radial expansion balloon and/or the movable distal bushing may occur, resulting in undesired twisting of the plurality of axial expansion chambers relative to each other, affecting the efficiency of the device. The use of three or more tubular axial expansion chambers, in each of the first unit and second unit, avoids this undesired twisting movements thereof.

In one example, the axial actuator may comprise three wires each attached between the movable support bushing and the movable distal bushing. This configuration of wires provides a high stability to the axial actuator.

According to one example, each of the fixed radial expansion balloon and the movable radial expansion balloon may comprise, respectively, a toroidal shaped body.

In one preferred example, each of the fixed radial expansion balloon and the movable radial expansion balloon may comprise respectively an external surface provided with a plurality of protrusions configured to contact the inner wall of the digestive tract. The use of such protrusions improves the adherence effect to the inner wall of the digestive tract.

In one example, the device may comprise a plurality of tubes individually connected to the radial expansion balloons and to the first and second units of axial expansion chambers to supply a pressurized fluid thereto.

In one preferred example, the device further may comprise a fixed proximal bushing to be immobilized to a proximal portion of the endoscope, and configured for collecting and supporting the plurality of tubes on the endoscope.

According to another aspect, the present disclosure also provides a system for assisting translational movement of an endoscope through the digestive tract, comprising
- a device for assisting translational movement of an endoscope as disclosed herein;
- a fluid supply unit in fluid communication with the device for supplying a pressurized fluid to the radial expansion balloons and the first and second units of axial expansion chambers; and
- a control unit for controlling the automated and coordinated operation of the fluid supply unit.

In examples, the control unit is configured to operate the fluid supply unit to perform, from an initial condition in which the fixed radial expansion balloon is deflated and the movable radial expansion balloon is inflated, and the movable radial expansion balloon is in the distal position, the following steps:
- deflating the second unit of axial expansion chambers and inflating the first unit of axial expansion chambers, to displace the movable radial expansion balloon in a proximal direction from the distal position to the proximal position;
- inflating the fixed radial expansion balloon to contact an inner wall of the digestive tract;
- deflating the movable radial expansion balloon;
- deflating the first unit of axial expansion chambers and inflating the second unit of axial expansion chambers, to displace the movable radial expansion balloon in a distal direction from the proximal position up to the distal position;
- inflating the movable radial expansion balloon to contact an inner wall of the digestive tract;
- deflating the fixed radial expansion balloon to return to the initial condition.

Furthermore, the control unit may be configured to operate the fluid supply unit to repeat the same steps, thus performing a number of cycles, until a further user input is received.

### BRIEF DESCRIPTION OF THE DRAWINGS

Non-limiting examples of the present disclosure will be described in the following, with reference to the appended drawings, in which:
Figure 1 is a perspective view of a device for assisting translational movement of an endoscope through the digestive tract, according to one example of the present disclosure;
Figure 2 is a perspective exploded view of the device shown in figure 1;
Figure 3 is a perspective view of an example radial expansion balloon of the device of the present disclosure;
Figure 4 is a perspective view of an example movable distal bushing of the device of the present disclosure;
Figure 5 is a perspective view of an example fixed intermediate bushing of the device of the present disclosure;
Figure 6 is a perspective view of an example movable support bushing for supporting the movable radial expansion balloon of the device of the present disclosure;
Figure 7 is a perspective view of an example fixed support bushing for supporting the fixed radial expansion balloon of the device of the present disclosure;
Figure 8 is a perspective view of an example fixed proximal bushing of the device of the present disclosure;
Figure 9 is a schematic representation of the operation method of the device of figure 1;
Figure 10a is a detail view of the stage S2 in figure 9, with the digestive tract illustrated schematically, when the movable radial expansion balloon is placed in the distal position, and in its inflated state to contact the inner wall of the digestive tract;
Figure 10b is a detail view of the stage S3 in figure 9, with the digestive tract illustrated schematically, when the movable radial expansion balloon is moved backward up to the proximal position, for retracting a portion of the digestive tract on the endoscope;
Figure 11 is a schematic diagram of a system for assisting translational movement of an endoscope through the digestive tract, according to one example of the present disclosure;
Figure 12 is a perspective view of a device according to an alternative example of the present disclosure; and
Figure 13 is a schematic representation of the operation method of the device of figure 12.

### DETAILED DESCRIPTION OF EXAMPLES

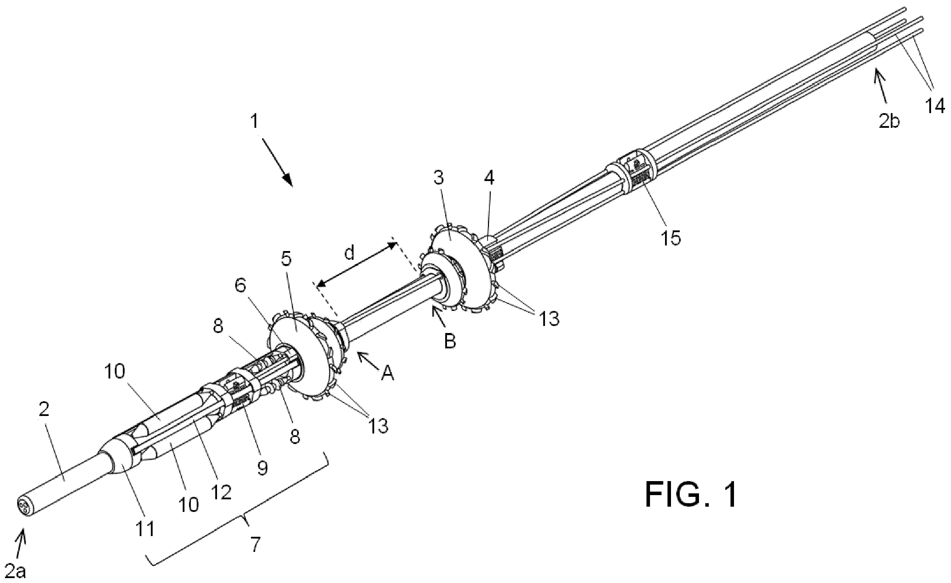

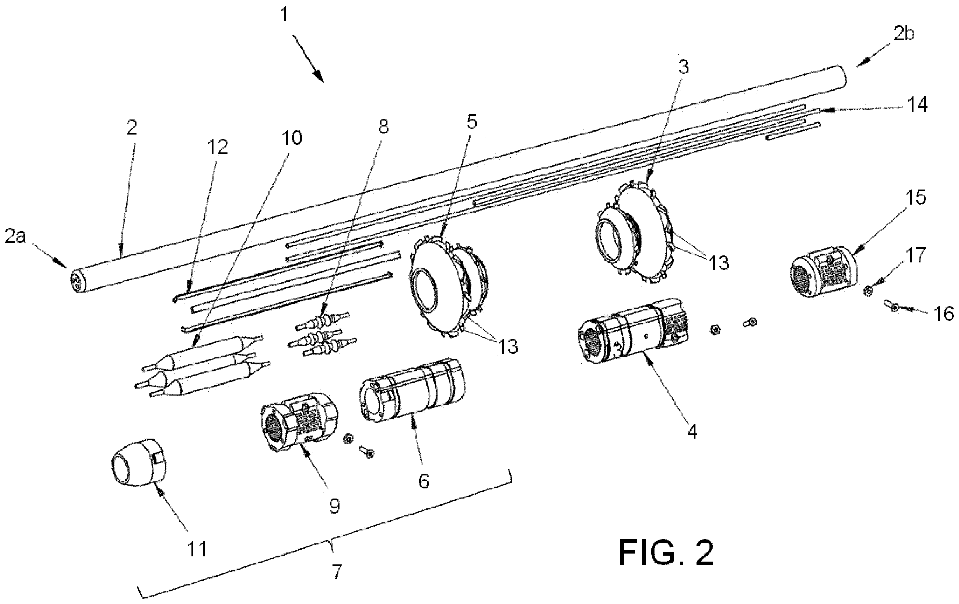

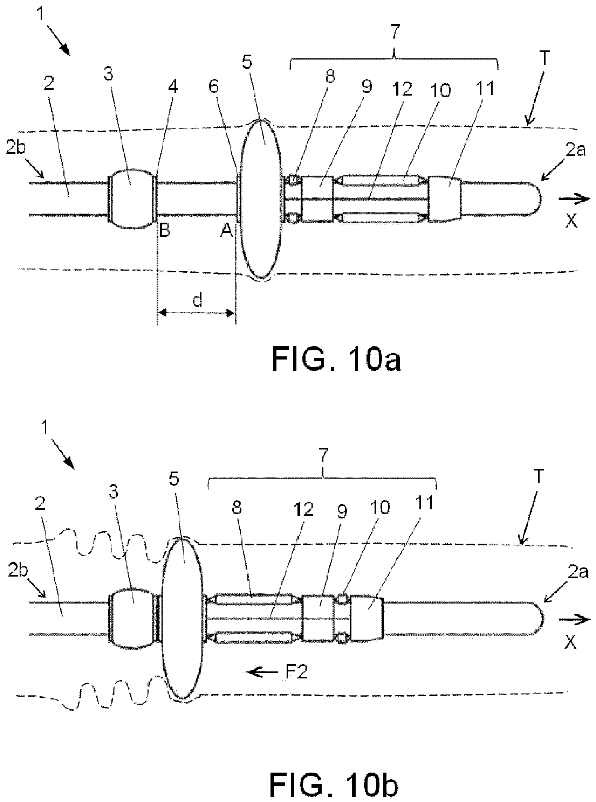

Figures 1 and 2 shown an example of a device 1 of the present disclosure for assisting translational movement of an endoscope 2 through the digestive tract T (illustrated schematically in dashed lines in figures 10a and 10b).

In the example of figures 1 and 2, the device 1 comprises:
- a fixed radial expansion balloon 3 mounted on a fixed support bushing 4 to be immobilized on the endoscope 2;
- a movable radial expansion balloon 5 mounted on a movable support bushing 6 to be mounted floating on the endoscope 2;
   wherein the fixed radial expansion balloon 3 and the movable radial expansion balloon 5 are configured to contact an inner wall of the digestive tract T in an expanded position; and
- an axial actuator 7 to be attached between the movable radial expansion balloon 5 and a distal end 2a of the endoscope 2, wherein the axial actuator 7 comprises:
   - a first unit of axial expansion chambers 8 attached by respective opposite ends between the movable support bushing 6 of the movable radial expansion balloon 5 and a fixed intermediate bushing 9, the fixed intermediate bushing 9 to be immobilized on the endoscope 2;
   - a second unit of axial expansion chambers 10 attached by respective opposite ends between the fixed intermediate bushing 9 and a movable distal bushing 11, the movable distal bushing 11 to be mounted floating on the endoscope 2 in a position distal with respect to the first and second units of axial expansion chambers 8, 10; and
   - a wire 12, e.g. a rigid wire, attached by respective opposite ends between the movable support bushing 6 of the movable radial expansion balloon 5 and the movable distal bushing 11.

The first unit of axial expansion chambers 8 and the second unit of axial expansion chambers 10 are configured to be synchronously extended and contracted to move axially the movable support bushing 6 of the movable radial expansion balloon 5 with alternate forward and backward movements, along a working distance "d" between
- a distal position A, wherein the first unit of axial expansion chambers 8 is contracted and the second unit of axial expansion chambers 10 is extended (see e.g. figure 10a), and
- a proximal position B, wherein the first unit of axial expansion chambers 8 is extended and the second unit of axial expansion chambers 10 is contracted (see e.g. figure 10b).

The operation method of the device 1 will be explained below in more detail.

The working distance "d" of the movable radial expansion balloon 5 may be comprised between 3 and 15 cm, for example between 5 and 10 cm, and preferably may be approximately 6 cm. Therefore, a large portion of the digestive tract T may be retracted on the endoscope 2 in each cycle.

In the present example, each of the first unit of axial expansion chambers 8 and the second unit of axial expansion chambers 10 may comprise three axial expansion chambers arranged parallel to each other and around the endoscope 2. As shown in figures 1 and 2, each axial expansion chamber 8, 10 may comprise a tubular shaped body.

The axial expansion chambers 8, 10 may be made of a biocompatible material, e.g. nylon or PET.

Furthermore, the material used for the axial expansion chambers may be non-compliant. It is understood that a "non-compliant" material, when used in an axial expansion chamber as described herein, allows an accuracy pressure control since a non-compliant chamber does not increase significantly in diameter when inflated to high pressures. As a result, an accurate extension of the axial expansion chambers is guaranteed.

As explained above, on the one hand, the tubular shape of the axial expansion chambers 8, 10 provides a greater propulsion force for moving the movable radial expansion balloon 5, backwards and forwards, according to the coordinated expansion and contraction movements of the respective first unit 8 and second unit 10 of axial expansion chambers. On the other hand, the use of three or more axial expansion chambers results in an optimal design since it avoids the undesired twisting movements during the advance of the endoscope though the digestive tract.

In the present example, the axial actuator 7 comprises three wires 12 each attached between the movable support bushing 6 and the movable distal bushing 11, and the three wires 12 are arranged spaced apart from each other around the endoscope 2, thus providing a high stability to the axial actuator 7. Moreover, the wires 12 may be made of a biocompatible material, e.g. nylon.

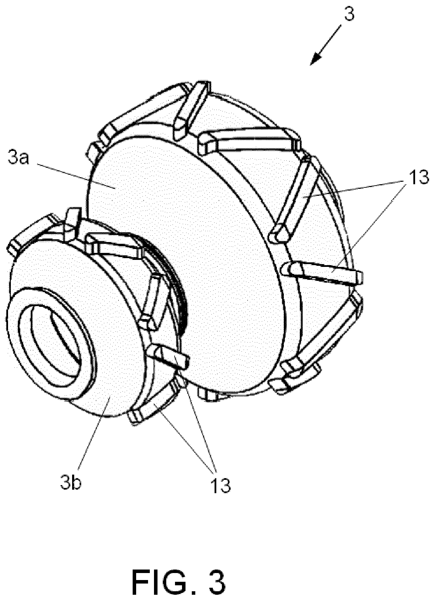

In the present example, each of the fixed radial expansion chamber 3 and the movable radial expansion chamber 5 may comprise, respectively, a toroidal shaped body. As can be seen in figure 3, the toroidal shaped body may be configured with a main toroidal body 3a joined to an additional toroidal body 3b with a smaller diameter placed adjacent and in fluid communication to each other. This configuration prevents a portion of the digestive tract T to be undesirable caught between the movable radial expansion balloon 5 and the fixed radial expansion balloon 3 when the digestive tract T is being retracted.

Each of the fixed radial expansion balloon 3 and the movable radial expansion balloon 5 may be respectively made of biocompatible material, e.g. a biocompatible silicone.

Furthermore, the material used for the radial expansion balloons may be compliant. It is understood that a "compliant" material, when used in a radial expansion balloon as described herein, allows an accuracy volume control since a compliant balloon increases significantly in diameter when inflated to low pressures, thus providing an optimal contact of the radial expansion balloons to the inner wall of the digestive tract.

Each of the fixed radial expansion balloon 3 and the movable radial expansion balloon 5 further may comprise respectively an external surface provided with a plurality of protrusions 13 configured to contact the inner wall of the digestive tract T, thus improving its adherence effect. As can be seen in figure 3, for example, each protrusion 13 may comprise an elongate rectangular shaped body, and the protrusions 13 may be distributed according to a zig zag pattern, or another suitable configuration.

As shown in figures 1 and 2, the device 1 comprises a plurality of tubes 14 individually connected to the radial expansion balloons 3, 5 and to the first and second units of axial expansion chambers 8, 10 to supply a pressurized fluid thereto.

In the present example, the device comprises a fixed proximal bushing 15 to be immobilized to a proximal portion 2b of the endoscope 2, and configured for collecting and supporting the plurality of tubes 14 on the endoscope 2.

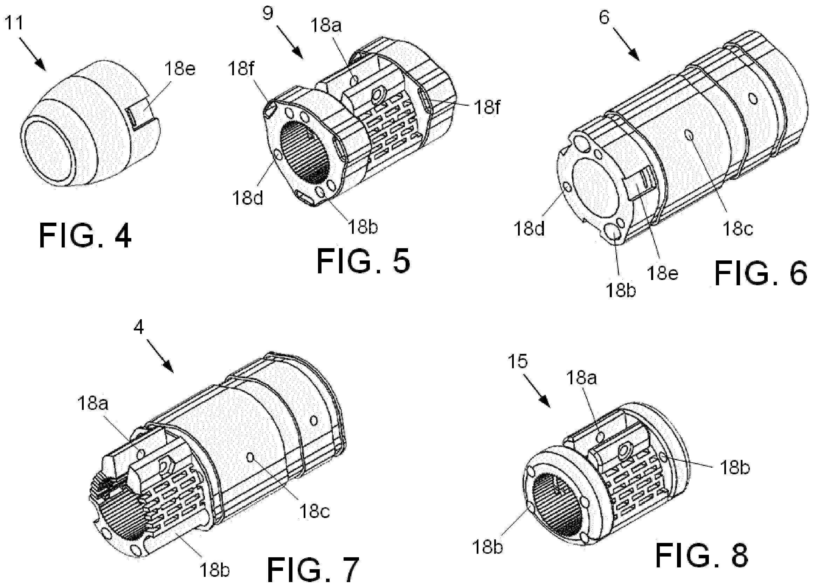

Figures 4 to 8 show an example of the respective bushings described herein, as explained below.

The movable distal bushing 11 (see figure 4) may comprise a tapered annular tip. This configuration provides a fluid-dynamic shape for facilitating the access through the digestive tract T.

The fixed support bushing 4, the fixed intermediate bushing 9 and fixed proximal bushing 15 (see figures 7, 5 and 8 respectively) comprise through holes 18a provided for receiving screws16 and nuts 17 (visible in figure 2) for its tightening and fixation to the endoscope 2. Alternatively, other suitable known fixation elements may be used.

The fixed proximal bushing 15, the fixed support bushing 4, the movable support bushing 6 and the fixed intermediate bushing 9 (see figures 8, 7, 6 and 5 respectively) comprise through holes 18b configured for providing the passage of the respective tubes 14.

The fixed support bushing 4 and the movable support bushing 6 (see figures 7 and 6 respectively) further comprise through holes 18c to direct the pressurized fluid flow (e.g. air) from the respective tubes 14 (visible e.g. in figure 1) to the fixed radial expansion balloon 3 and the movable radial expansion balloon 5, respectively.

The movable distal bushing 11, the fixed intermediate bushing 9 and the movable support bushing 6 (see figures 4, 5 and 6 respectively) comprise blind holes 18d for attaching the respective ends of the corresponding axial expanding chambers 8, 10.

Furthermore, the movable distal bushing 11 and the movable support bushing 6 (see figures 4 and 6 respectively) comprise slots 18e for attaching the respective ends of the wires 12, and the fixed intermediate bushing 9 (see figure 5) also comprises through holes 18f aligned with said slots 18e to provide the passage and support of the wires 12.

The different bushings 4, 6, 9, 11 and 15 described here may be made of a biocompatible material, e.g. a resin material.

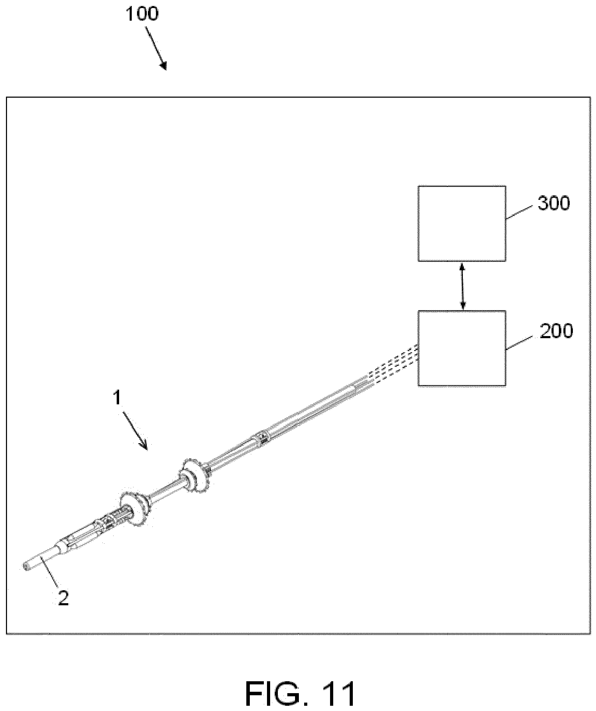

As shown in figure 11, the present disclosure also provides a system 100 for assisting translational movement of an endoscope 2 through the digestive tract T, comprising
- a device 1 for assisting translational movement of an endoscope 2 as disclosed herein;
- a fluid supply unit 200 in fluid communication with the device 1 for supplying a pressurized fluid to the radial expansion balloons 3, 5 and to the first and second units of axial expansion chambers 8, 10; and
- a control unit 300 for controlling the automated and coordinated operation of the fluid supply unit 200.

The fluid supply unit 200 may be pneumatic (using air o gas as a fluid), or hydraulic (using a liquid fluid).

According to an example, the supply unit 200 may comprise a plurality of electrovalves connected to the respective tubes 14, for enabling the activation and desactivation of the fluid flow to the device 1. Thanks to the activation and deactivation of the electrovalves a movement sequence is established.

In one example, the control unit 300 may comprise a microcontroller and a communication interface (e.g. a data display screen and a keyboard, a tactile screen, etc).

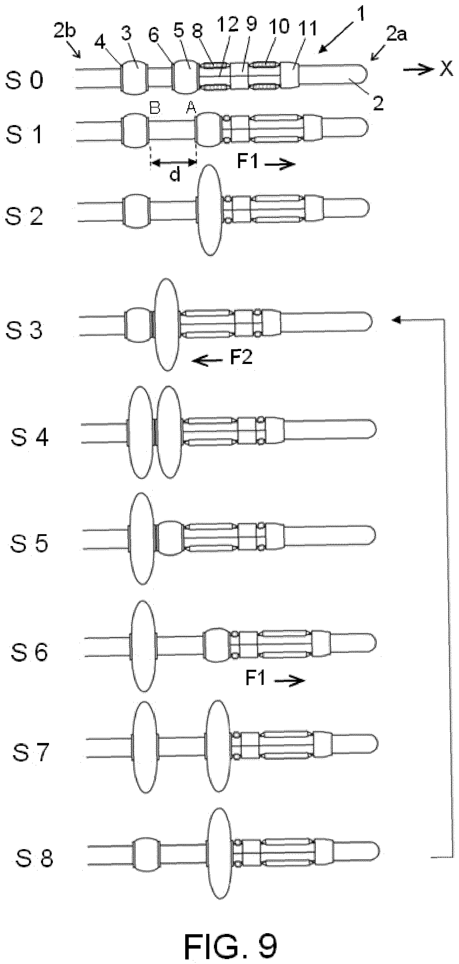

Figure 9 shows a schematic representation of a method for implementing the translational movement of an endoscope through the digestive tract, using the device 1 (see e.g. figures 1 and 2) according to examples as previously described.

Such a method may be implemented by a system 100 as described above, in which the control unit 300 operates the fluid supply unit 200 to perform the steps of the method. The method may be started when a user, e.g. a doctor, inputs an order through the communication interface.

As shown in figure 9, this example of a method for implementing the translational movement of an endoscope 2 through the digestive tract T comprises at least the following steps:
- in step S0, inserting the endoscope 2 in the digestive tract T with both fixed 3 and movable 5 radial expansion balloons, and with both first unit 8 and second unit 10 of axial expansion chambers without pressure in a rest initial position.
- in step S1, deflating the first unit of axial expansion chambers 8 and inflating the second unit of axial expansion chambers 10, thus providing a forward propulsion force F1 capable of displacing the movable distal bushing 11 forward (i.e., in distal direction, towards the distal end 2a of the endoscope 2 and in the advance direction X of the endoscope 2), and in turn the wire 12 pulls the movable radial expansion balloon 5 forwards from the rest initial position up to a distal position A, adjacent to the fixed intermediate bushing 9. The deflation of the first unit of axial expansion chambers 8 and the inflation of the second unit of axial expansion chambers 10 may be simultaneous.
- in step S2, inflating the movable radial expansion balloon 5 to contact an inner wall of the digestive tract T (also visible in Figure 10a).
This brings the system to an initial condition from which the following steps are performed, in order to retract a portion of the digestive tract T on the endoscope 2:
- in step S3, deflating the second unit of axial expansion chambers 10 and inflating the first unit of axial expansion chambers 8, thus providing a backward propulsion force F2 capable of displacing the movable radial expansion balloon 5 backwards (i.e., in proximal direction, opposite to the advance direction X of the endoscope 2), along a working distance "d" from the distal position A up to a proximal position B, adjacent to the fixed radial expansion balloon 3, and in turn the wire 12 pulls the movable distal bushing 11 backwards. The backward displacement of the movable radial expansion chamber 5 provides the retraction of a portion of the digestive tract T whereto it was contacted (also visible in Figure 10b). The deflation of the second unit of axial expansion chambers 10 and the inflation of the first unit of axial expansion chambers 8 may be simultaneous.
- in step S4, inflating the fixed radial expansion balloon 3 to contact an inner wall of the digestive tract T.
- in step S5, deflating the movable radial expansion balloon 5, while keeping the portion of the digestive tract T retracted behind the inflated fixed radial expansion balloon 3, thus achieving indirectly the effective advance of the endoscope 2 through the digestive tract T.
- In step S6, deflating the first unit of axial expansion chambers 8 and inflating the second unit of axial expansion chambers 10, thus providing a forward propulsion force F1 capable of displacing the movable distal bushing 11 forward, and in turn the wire 12 pulls the movable radial expansion balloon 5 forwards from along the working distance "d" from the proximal position B up to the distal position A.
- In step S7, inflating again the movable radial expansion balloon 5 to contact an inner wall of the digestive tract T.
- In step S8, deflating the fixed radial expansion balloon 3 to return to the initial condition, from which steps S3 to S8 may be repeated.

Steps S3 to S8 are then repeated, thus performing a number of cycles, until the user indicates, with an order inputted through the communication interface, that the process has to be stopped or paused.

Therefore, by repeating successively the steps S3 to S8, it is possible to retract the digestive tract T on the endoscope 2 and indirectly move the endoscope 2 forwards through the digestive tract T.

Figures 10a and 10b are respectively detail views of the stages S2 and S3 in figure 9, showing also the digestive tract T (illustrated in dashed lines) for clarity showing the portion of digestive tract T retracted on the endoscope 2.

As explained, the alternate and coordinate movement of the first unit of axial expansion chambers 8 and the second unit of axial expansion chambers 10, provides a significant backward force F2 for retracting a large portion of the digestive tract T on the endoscope 2, thus providing a relevant advance distance of the endoscope 2 through the digestive tract T, in each cycle, and especially to overcome the force exerted by the mesentery. On the other hand, a significant forward force F1 to return the movable radial expansion balloon 5 to the distal position A is achieved, in each cycle.

It should be mentioned that the automated and coordinated movement of both fixed 3 and movable 5 radial expansion balloons, and both first unit 8 and second unit 10 of axial expansion chambers, that facilitates the retraction of the digestive tract, could even be programmed to carry out an inverse movement for expanding the digestive tract in the event of need of the digestive tract or blocking thereof.

The doctor may control the advancement of the endoscope 2 in real time, through a camera incorporated in the distal end 2a of the endoscope 2, so that after several cycles of intestinal retraction (for example between 5 and 30 successive retraction cycles), if any difficulty in advancing the endoscope 2 is observed, the doctor can manually move the endoscope 2 in the direction of advance X when necessary, for example, when the endoscope 2 is at a point in the digestive tract T with a pronounced curvature that hinders its advancement. Furthermore, the doctor can also pull the endoscope 2 in the opposite direction to X to facilitate the retraction and achieve a certain rectification of the digestive tract T that remains to be explored in order to allow the endoscope 2 to continue advancing.

In this example of the device 1 (see e.g. figures 1 and 9), the fixed radial expansion balloon 3 is mounted on a fixed support bushing 4 placed in a position adjacent to the proximal position B of the working distance "d".

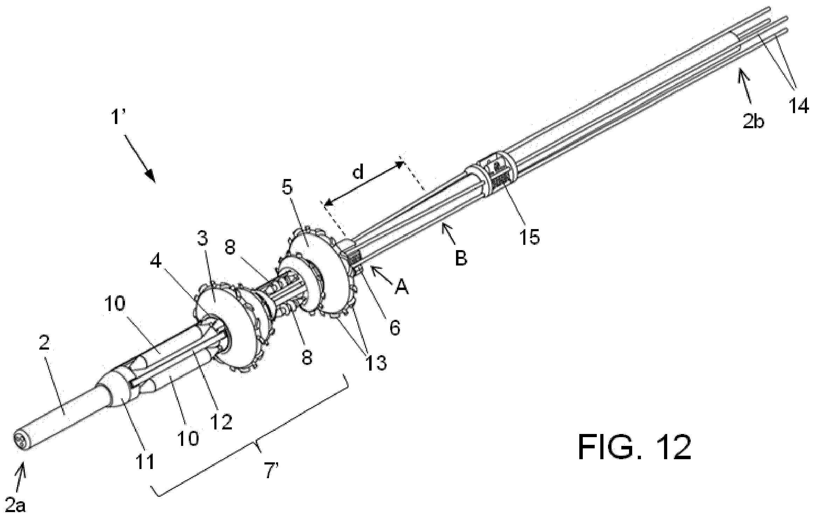

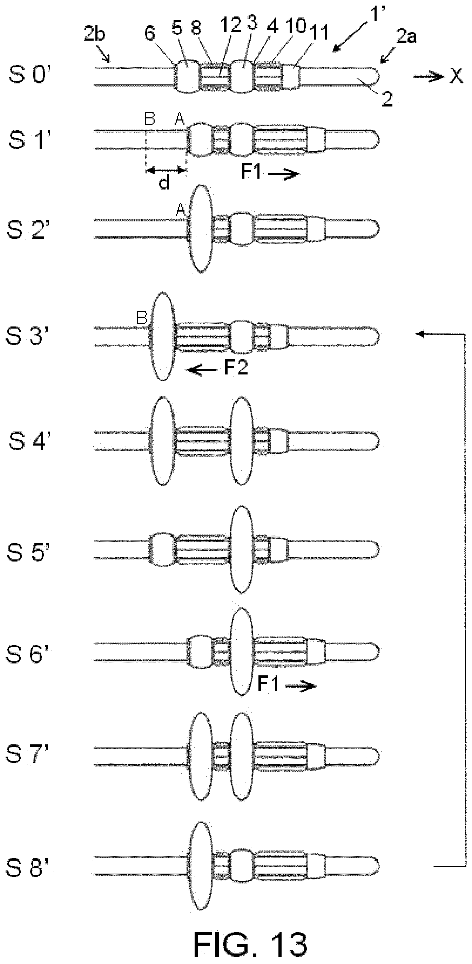

Figures 12 and 13 shown an alternate example of a device 1' of the present disclosure for assisting translational movement of an endoscope 2 through the digestive tract T. For clarity, the common features of the device 1' with respect to the device 1 of the previous example (see e.g. figures 1 and 9) are indicated using the same referral numeral.

As can be seen in figure 12, the device 1' comprises an axial actuator 7' which differs from the axial actuator 7 of the device 1 of the previous example. In particular, the fixed radial expansion balloon 3 is mounted on a fixed support bushing 4 placed in a position between the first unit of axial expansion chambers 8 and the second unit of axial expansion chambers 10. Therefore, in this alternative example, the fixed intermediate bushing 9 of the device 1 of the previous example is omitted and replaced by the mentioned fixed support bushing 4 of the device 1'.

Thus, the fixed support bushing 4 is configured for supporting the fixed radial expansion balloon 3 and also adapted for acting as an intermediate bushing for attaching the corresponding ends of both first and second units of axial expansion chambers 8, 10.

Figure 13 shows a schematic representation of a method for implementing the translational movement of an endoscope through the digestive tract, using the device 1' of this alternative example. This method comprises at least the following steps:
- in step S0', inserting the endoscope 2 in the digestive tract T with both fixed 3 and movable 5 radial expansion balloons, and with both first unit 8 and second unit 10 of axial expansion chambers without pressure in a rest initial position.
- in step S1', deflating the first unit of axial expansion chambers 8 and inflating the second unit of axial expansion chambers 10, thus providing a forward propulsion force F1 capable of displacing the movable distal bushing 11 forward, and in turn the wire 12 pulls the movable radial expansion balloon 5 forwards from the rest initial position up to a distal position A. The deflation of the first unit of axial expansion chambers 8 and the inflation of the second unit of axial expansion chambers 10 may be simultaneous.
- in step S2', inflating the movable radial expansion balloon 5 to contact an inner wall of the digestive tract T.

This brings the system to an initial condition from which the following steps are performed, in order to retract a portion of the digestive tract T on the endoscope 2:
- in step S3', deflating the second unit of axial expansion chambers 10 and inflating the first unit of axial expansion chambers 8, thus providing a backward propulsion force F2 capable of displacing the movable radial expansion balloon 5 backwards, along a working distance "d" from the distal position A up to a proximal position B, adjacent to the fixed radial expansion balloon 3, and in turn the wire 12 pulls the movable distal bushing 11 backwards. The backward displacement of the movable radial expansion chamber 5 provides the retraction of a portion of the digestive tract T whereto it was contacted. The deflation of the second unit of axial expansion chambers 10 and the inflation of the first unit of axial expansion chambers 8 may be simultaneous.
- in step S4', inflating the fixed radial expansion balloon 3 to contact an inner wall of the digestive tract T.
- in step S5', deflating the movable radial expansion balloon 5, while keeping the portion of the digestive tract T retracted behind the inflated fixed radial expansion balloon 3, thus achieving indirectly the effective advance of the endoscope 2 through the digestive tract T.
- In step S6', deflating the first unit of axial expansion chambers 8 and inflating the second unit of axial expansion chambers 10, thus providing a forward propulsion force F1 capable of displacing the movable distal bushing 11 forward, and in turn the wire 12 pulls the movable radial expansion balloon 5 forwards from along the working distance "d" from the proximal position B up to the distal position A.
- In step S7', inflating again the movable radial expansion balloon 5 to contact an inner wall of the digestive tract T.
- In step S8', deflating the fixed radial expansion balloon 3 to return to the initial condition, from which steps S3 to S8 may be repeated.

Steps S3' to S8' are then repeated, thus performing a number of cycles, until the user indicates, with an order inputted through the communication interface, that the process has to be stopped or paused.

It is noted that such a method using the device 1' may also be implemented by the system 100 as described above (see figure 11).

Although only a number of examples have been disclosed herein, other alternatives, modifications, uses and/or equivalents thereof are possible. Furthermore, all possible combinations of the described examples are also covered. Thus, the scope of the present disclosure should not be limited by particular examples, but should be determined only by a fair reading of the claims that follow. If reference signs related to drawings are placed in parentheses in a claim, they are solely for attempting to increase the intelligibility of the claim, and shall not be construed as limiting the scope of the claim.

## Claims

1. A device (1, 1') for assisting translational movement of an endoscope (2) through the digestive tract (T), comprising:
- a fixed radial expansion balloon (3) mounted on a fixed support bushing (4) to be immobilized on the endoscope (2);
- a movable radial expansion balloon (5) mounted on a movable support bushing (6) to be mounted floating on the endoscope (2);
wherein the fixed radial expansion balloon (3) and the movable radial expansion balloon (5) are configured to contact an inner wall of the digestive tract (T) in an expanded position; and
- an axial actuator (7, 7') to be attached between the movable radial expansion balloon (5) and a distal end (2a) of the endoscope (2), wherein the axial actuator (7) comprises:
- a first unit of axial expansion chambers (8) attached at one end to the movable support bushing (6) of the movable radial expansion balloon (5);
- a second unit of axial expansion chambers (10) attached at one end to a movable distal bushing (11), the movable distal bushing (11) to be mounted floating on the endoscope (2) in a position distal with respect to the first and second units of axial expansion chambers (8, 10); and
- a wire (12) attached by respective opposite ends between the movable support bushing (6) of the movable radial expansion balloon (5) and the movable distal bushing (11);
and wherein the first unit of axial expansion chambers (8) and the second unit of axial expansion chambers (10) are configured to be synchronously extended and contracted to move axially the movable support bushing (6) of the movable radial expansion balloon (5) with alternate forward and backward movements, along a working distance (d) between
- a distal position A, wherein the first unit of axial expansion chambers (8) is contracted and the second unit of axial expansion chambers (10) is extended, and
- a proximal position B, wherein the first unit of axial expansion chambers (8) is extended and the second unit of axial expansion chambers (10) is contracted.

2. The device (1) according to claim 1, further comprising a fixed intermediate bushing (9) arranged between the first and second units of axial expansion chambers (8, 10), and to be immobilized on the endoscope (2), wherein
- the first unit of axial expansion chambers (8) is attached between the movable support bushing (6) and the fixed intermediate bushing (9); and
- the second unit of axial expansion chambers (10) is attached between the fixed intermediate bushing (9) and the movable distal bushing (11).

3. The device (1') according to claim 1, wherein
- the first unit of axial expansion chambers (8) is attached between the movable support bushing (6) and the fixed support bushing (4); and
- the second unit of axial expansion chambers (10) is attached between the fixed support bushing (4) and the movable distal bushing (11).

4. The device (1, 1') according to any of preceding claims, wherein the working distance (d) of the movable radial expansion balloon (5) is comprised between 3 and 15 cm.

5. The device (1, 1') according to claim 4, wherein the working distance (d) of the movable radial expansion balloon (5) is comprised between 5 and 10 cm.

6. The device (1, 1') according to any of preceding claims, wherein each axial expansion chamber of the first unit (8) and the second unit (10) comprises a tubular shaped body.

7. The device (1, 1') according to any of preceding claims, wherein each of the first unit of axial expansion chambers (8) and the second unit of axial expansion chambers (10) comprise three axial expansion chambers arranged parallel to each other and around the endoscope (2).

8. The device (1, 1') according to any of preceding claims, wherein the axial actuator (7, 7') comprises three wires (12) each attached between the movable support bushing (6) and the movable distal bushing (11).

9. The device (1, 1') according to any of preceding claims, wherein each of the fixed radial expansion balloon (3) and the movable radial expansion balloon (5) comprises, respectively, a toroidal shaped body.

10. The device (1, 1') according to any of preceding claims, wherein each of the fixed radial expansion balloon (3) and the movable radial expansion balloon (5) comprises respectively an external surface provided with a plurality of protrusions (13) configured to contact the inner wall of the digestive tract (T).

11. The device (1, 1') according to any of preceding claims, comprising a plurality of tubes (14) individually connected to the radial expansion balloons (3, 5) and to the first and second units of axial expansion chambers (8, 10) to supply a pressurized fluid thereto.

12. The device (1, 1') according to claim 11, further comprising a fixed proximal bushing (15) to be immobilized to a proximal portion (2b) of the endoscope (2), and configured for collecting and supporting the plurality of tubes (14) on the endoscope (2).

13. A system (100) for assisting translational movement of an endoscope (2) through the digestive tract (T), comprising
- a device (1, 1') for assisting translational movement of an endoscope (2) according to any of preceding claims;
- a fluid supply unit (200) in fluid communication with the device (1, 1') for supplying a pressurized fluid to the radial expansion balloons (3, 5) and to the first and second units of axial expansion chambers (8, 10); and
- a control unit (300) for controlling the automated and coordinated operation of the fluid supply unit (200).

14. A system (100) according to claim 13, wherein the control unit (300) is configured to operate the fluid supply unit (200) to perform, from an initial condition in which the fixed radial expansion balloon (3) is deflated and the movable radial expansion balloon (5) is inflated, and the movable radial expansion balloon is in the distal position (A), the following steps:
- deflating the second unit of axial expansion chambers (10) and inflating the first unit of axial expansion chambers (8), to displace the movable radial expansion balloon (5) in a proximal direction from the distal position (A) to the proximal position (B);
- inflating the fixed radial expansion balloon (3) to contact an inner wall of the digestive tract (T);
- deflating the movable radial expansion balloon (5);
- deflating the first unit of axial expansion chambers (8) and inflating the second unit of axial expansion chambers (10), to displace the movable radial expansion balloon (5) in a distal direction from the proximal position (B) up to the distal position (A);
- inflating the movable radial expansion balloon (5) to contact an inner wall of the digestive tract (T);
- deflating the fixed radial expansion balloon (3) to return to the initial condition.

15. A system (100) according to claim 14, wherein the control unit (300) is configured to operate the fluid supply unit (200) to repeat the same steps until a further user input is received.
